# EUROPEAN PATENT SPECIFICATION

(11) **EP 1 996 063 B1**
(45) Date of publication and mention of the grant of the patent: **03.07.2019**
(21) Application number: 07759199.8
(22) Date of filing: 22.03.2007
(51) Int. Cl.: A61B 1/00, A61M 25/00, G01D 5/353, G02B 6/02, G01B 11/16, A61B 5/06, A61B 1/005, A61M 25/01, G02B 23/26, A61B 34/30, A61B 34/00, A61B 34/20

(54) **FIBER OPTIC INSTRUMENT SENSING SYSTEM**
ERFASSUNGSSYSTEM MIT EINEM FASEROPTISCHEN INSTRUMENT
INSTRUMENT DE DETECTION MEDICAL A FIBRE OPTIQUE

(30) Priority: 22.03.2006 US 785001 P; 31.03.2006 US 788176 P
(43) Date of publication of application: 03.12.2008
(60) Divisional application: 19173731.1
(73) Proprietor: Koninklijke Philips N.V., 5656 AE Eindhoven (NL)
(72) Inventor: SCHLESINGER, Randall, L., San Mateo, CA 94403 (US); KING, Toby, St.John, Hampshire S031 9FG (GB); LUNDMARK, David, Los Altos, CA 94024 (US)
(74) Representative: Lecomte & Partners
(86) International application number: PCT/US2007/064728
(87) International publication number: WO 2007/109778

(56) References cited:
- WO-A-01/33165
- WO-A-2005/087128
- US-A1- 2001 021 843
- US-A1- 2004 165 810
- US-A1- 2006 013 523
- US-B1- 6 611 700
- US-B1- 6 716 178

## Description

### FIELD OF INVENTION

The invention relates generally to medical instruments, such as elongate steerable instruments for minimally-invasive intervention or diagnosis, and more particularly to a system and apparatus for sensing or measuring the position and/or temperature at one or more distal positions along the elongate steerable medical instrument.

### BACKGROUND

Currently known minimally invasive procedures for diagnosis and treatment of medical conditions use elongate instruments, such as catheters or more rigid arms or shafts, to approach and address various tissue structures within the body. For various reasons, it is highly valuable to be able to determine the 3-dimensional spatial position of portions of such elongate instruments relative to other structures, such as the operating table, other instruments, or pertinent tissue structures. It is also valuable to be able to detect temperature at various locations of the instrument. Conventional technologies such as electromagnetic position sensors, available from providers such as the Biosense Webster division of Johnson & Johnson, Inc., or conventional thermocouples, available from providers such as Keithley Instruments, Inc., may be utilized to measure 3-dimensional spatial position or temperature, respectively, but may be limited in utility for elongate medical instrument applications due to hardware geometric constraints, electromagnetivity issues, etc.

There is a need for an alternative technology to facilitate the execution of minimally-invasive interventional or diagnostic procedures while monitoring 3-dimensional spatial position and/or temperature.

It is well known that by applying the Bragg equation (wavelength = 2^{∗}d^{∗}sin(theta)) to detect wavelength changes in reflected light, elongation in a diffraction grating pattern
positioned longitudinally along a fiber or other elongate structure maybe be determined. Further, with knowledge of thermal expansion properties of fibers or other structures which carry a diffraction grating pattern, temperature readings at the site of the diffraction grating may be calculated.

Socalled "fiberoptic Bragg grating" ("FBG") sensors or components thereof, available from suppliers such as Luna Innovations, Inc., of Blacksburg, Virginia, Micron Optics, Inc., of Atlanta, Georgia, LxSix Photonics, Inc., of Quebec, Canada, and Ibsen Photonics A/S, of Denmark, have been used in various applications to measure strain in structures such as highway bridges and aircraft wings, and temperatures in structures such as supply cabinets. An objective of this invention is to measure strain and/or temperature at distal portions of a steerable catheter or other elongate medical instrument to assist in the performance of a medical diagnostic or interventional procedure.

WO 01/33165 A1 discloses an optical fiber navigation system according to the preamble of appended claim 1.

US 2006/0013523 A1 discloses a fiber optic position and shape sensing device. The device comprises optical fiber cores with Bragg gratings disposed within each fiber core. The fiber cores are affixed to an object for determining its position or shape.

US 6,611,700 B1 discloses a method and an apparatus for positioning a body for radiation using a position sensor. In particular, a glass fiber cable serving as a position sensor is surrounded by a sheath. Arranged within the sheath surrounding the glass fiber cable are one or more strips of a plastics material so that, due to a strip or the position of the strips relative to one another being shifted from outside, the glass fiber cable can be suitably curved.

US 2001/0021843 A1 discloses an elongate instrument body formed by a number of successively arranged rigid sections with respective successive sections being connected to one another via articulated joints which can be angled relative to one another. Fiber Bragg gratings may be position at the joints so that with the known geometry of the rigid sections the fiber Bragg gratings may detect the deformation of the joints.

US 2004/0165810 A1 discloses the application of a FBG sensor cable in an elongated flexible body. In particular, the sensor cable may have an outer diameter close to the inside diameter of the surrounding endoscopic biopsy channel such that placement of the sensor cable is not hindered by tightness.

### BRIEF DESCRIPTION OF THE DRAWINGS

The drawings illustrate the design and utility of illustrated embodiments of the invention, in which similar elements are referred to by common reference numerals.
Fig. 1 illustrates an example of an elongate instrument such as a conventional manually operated catheter.
Fig. 2 illustrates another example of an elongate instrument such as a robotically-driven steerable catheter.
Figs 3A-3C illustrate implementations of an optical fiber with Bragg gratings to an elongate instrument such as a robotically-steerable catheter.
Figs 4A-4D illustrate implementations of an optical fiber with Bragg gratings to an elongate instrument such as a robotically-steerable catheter.
Figs 5A-D illustrate implementation of an optical fiber with Bragg gratings to an elongate instrument as a robotically-steerable catheter.
Fig. 6 illustrates a cross sectional view of an elongate instrument such as a catheter including an optical fiber with Bragg gratings.
Fig. 7 illustrates a cross sectional view of an elongate instrument such as a catheter including a multi-fiber Bragg grating configuration.
Fig. 8 illustrates a cross sectional view of an elongate instrument such as a catheter including a multi-fiber Bragg grating configuration.
Figs 9A-9B illustrate top and cross sectional views of an elongate instrument such as a catheter having a multi-fiber structure with Bragg gratings.
Figs 10A-10B illustrate top and cross sectional views of an elongate instrument such as a catheter having a multi-fiber structure with Bragg gratings.
Figs 11A-11B illustrate top and cross sectional views of an elongate instrument such as a catheter having a multi-fiber structure with Bragg gratings.
Figs 12A-12H illustrate cross sectional views of elongate instruments with various fiber positions and configurations.
Fig. 13 illustrates an optical fiber sensing system with Bragg gratings.
Figs 14A-14B illustrates an optical fiber sensing system with Bragg gratings.
Figs 15A-15B illustrate optical fiber sensing system configurations with Bragg gratings
Figs 16A-16D illustrates integration of an optical fiber sensing system to a robotically-controlled guide catheter conifguration.
Figs 17A-17G illustrate integration of an optical fiber sensing system to a robotically-controlled sheath catheter configuration.
Fig. 18 illustrates a cross sectional view of a bundle of optical fiber within the working lumen of a catheter.

### SUMMARY OF THE INVENTION

The present invention is defined by appended claim 1.

### DETAILED DESCRIPTION OF THE ILLUSTRATED EMBODIMENTS

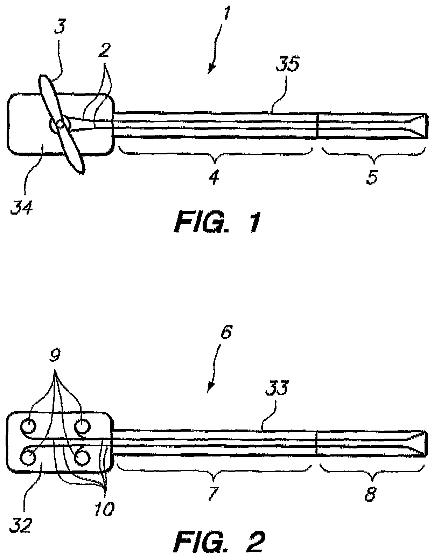

Referring to Figure 1, a conventional manually-steerable catheter (1) is depicted. Pullwires (2) may be selectively tensioned through manipulation of a handle (3) on the proximal portion of the catheter structure to make a more flexible distal portion (5) of the catheter bend or steer controllably. The handle (3) may be coupled, rotatably or slidably, for example, to a proximal catheter structure (34) which may be configured to be held in the hand, and may be coupled to the elongate portion (35) of the catheter (1). A more proximal, and conventionally less steerable, portion (4) of the catheter may be configured to be compliant to loads from surrounding tissues (for example, to facilitate passing the catheter, including portions of the proximal portion, through tortuous pathways such as those formed by the blood vessels), yet less steerable as compared with the distal portion (5).

Referring to Figure 2, a robotically-driven steerable catheter (6), similar to those described in detail in U.S. Patent Application serial number 11/176,598, is depicted. This catheter (6) has some similarities with the manually-steerable catheter (1) of Figure 1 in that it has pullwires (10) associated distally with a more flexible section (8) configured to steer or bend when the pullwires (10) are tensioned in various configurations, as compared with a less steerable proximal portion (7) configured to be stiffer and more resistant to bending or steering. The depicted embodiment of the robotically-driven steerable catheter (6) comprises proximal axles or spindles (9) configured to primarily interface not with fingers or the hand, but with an electromechanical instrument driver configured to coordinate and drive, with the help of a computer, each of the spindles (9) to produce precise steering or bending movement of the catheter (6). The spindles (9) may be rotatably coupled to a proximal catheter structure (32) which may be configured to mount to an electromechanical instrument driver apparatus, such as that described in the aforementioned U.S. Patent Application serial number 11/176,598, and may be coupled to the elongate portion (33) of the catheter (6).

Each of the embodiments depicted in Figures 1 and 2 may have a working lumen (not shown) located, for example, down the central axis of the catheter body, or may be without such a working lumen. If a working lumen is formed by the catheter structure, it may extend directly out the distal end of the catheter, or may be capped or blocked by the distal tip of the catheter. It is highly useful in many procedures to have precise information regarding the position of the distal tip of such catheters or other elongate instruments, such as those available from suppliers such as the Ethicon Endosurgery division of Johnson & Johnson, or Intuitive Surgical Corporation. The examples and illustrations that follow are made in reference to a robotically-steerable catheter such as that depicted in Figure 2, the same principles may be applied to other elongate instruments, such as the manually-steerable catheter depicted in Figure 1, or other elongate instruments, highly flexible or not, from suppliers such as the Ethicon Endosurgery division of Johnson & Johnson, Inc., or Intuitive Surgical, Inc.

The invention is defined by the appended claims. Subject matter referred to as embodiments and/or inventions which are not claimed are not part of the invention.

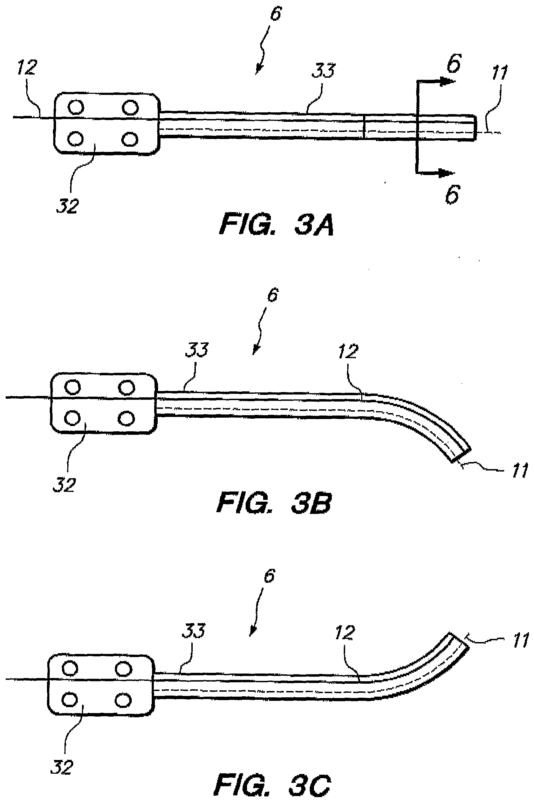

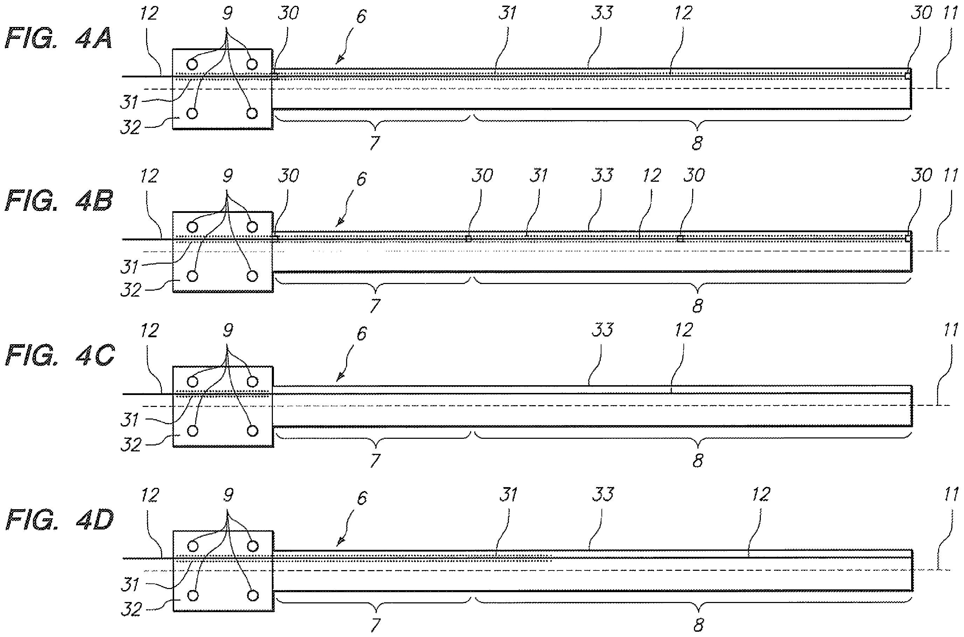

Referring to Figures 3A-3C, a robotically-steerable catheter (6) is depicted having an optical fiber (12) positioned along one aspect of the wall of the catheter (6). The fiber is not positioned coaxially with the neutral axis of bending (11) in the bending scenarios depicted in Figures 3B and 3C. Indeed, with the fiber (12) attached to, or longitudinally constrained by, at least two different points along the length of the catheter (6) body (33) and unloaded from a tensile perspective relative to the catheter body in a neutral position of the catheter body (33) such as that depicted in Figure 3A, the longitudinally constrained portion of the fiber (12) would be placed in tension in the scenario depicted in Figure 3B, while the longitudinally constrained portion of the fiber (12) would be placed in compression in the scenario depicted in Figure 3C. Such relationships are elementary to solid mechanics, but may be applied as described herein with the use of a Bragg fiber grating to assist in the determination of temperature and/or defection of an elongate instrument. Referring to Figures 4A-5D, several different embodiments are depicted. Referring to Figure 4A, a robotic catheter (6) is depicted having a fiber (12) deployed through a lumen (31) which extends from the distal tip of the distal portion (8) of the catheter body (33) to the proximal end of the proximal catheter structure (32). In one embodiment a broadband reference reflector (not shown) is positioned near the proximal end of the fiber in an operable relationship with the fiber Bragg grating wherein an optical path length is established for each reflector/grating relationship comprising the subject fiber Bragg sensor configuration; additionally, such configuration also comprises a reflectometer (not shown), such as a frequency domain reflectometer, to conduct spectral analysis of detected reflected portions of light waves.

Constraints (30) are provided to prohibit axial or longitudinal motion of the fiber (12) at the location of each constraint (30). Further constraints (30) constrain the position of the fiber (12) relative to the lumen (31) in the location of the constraints (30). For example, in one variation of the embodiment depicted in Figure 4A, the most distal constraint (30) may be configured to disallow longitudinal or axial movement of the fiber (12) relative to the catheter body (33) at the location of such constraint (30), while the more proximal constraint (30) may merely act as a guide to lift the fiber (12) away from the walls of the lumen (31) at the location of such proximal constraint (30). As shown in the embodiment depicted in Figure 4A, the lumen (31) in the region of the proximal catheter structure (32) is without constraints to allow for free longitudinal or axial motion of the fiber relative to the proximal catheter structure (32). Constraints configured to prohibit relative motion between the constraint and fiber at a given location may comprise small adhesive or polymeric welds, interference fits formed with small geometric members comprising materials such as polymers or metals, locations wherein braiding structures are configured with extra tightness to prohibit motion of the fiber, or the like. Constraints configured to guide the fiber (12) but to also allow relative longitudinal or axial motion of the fiber (12) relative to such constraint may comprise small blocks, spheres, hemispheres, etc defining small holes, generally through the geometric middle of such structures, for passage of the subject fiber (12).

The embodiment of Figure 4B is similar to that of Figure 4A, with the exception that there are two additional constraints (30) provided to guide and/or prohibit longitudinal or axial movement of the fiber (12) relative to such constraints at these locations. In another variation of the embodiment depicted in Figure 4B, the proximal and distal constraints (30) may be total relative motion constraints, while the two intermediary constraints (30) may be guide constraints configured to allow longitudinal or axial relative motion between the fiber (12) and such constraints at these intermediary locations, but to keep the fiber aligned near the center of the lumen (31) at these locations.

Referring to Figure 4C, an embodiment similar to those of Figures 4A and 4B is depicted, with the exception that entire length of the fiber that runs through the catheter body (33) is constrained by virtue of being substantially encapsulated by the materials which comprise the catheter body (33). In other words, while the embodiment of Figure 4C does have a lumen (31) to allow free motion of the fiber (12) longitudinally or axially relative to the proximal catheter structure (32), there is no such lumen defined to allow such motion along the catheter body (33), with the exception of the space naturally occupied by the fiber as it extends longitudinally through the catheter body (33) materials which encapsulate it.

Figure 4D depicts a configuration similar to that of Figure 4C with the exception that the lumen (31) extends not only through the proximal catheter structure (32), but also through the proximal portion (7) of the catheter body (33); the distal portion of the fiber (12) which runs through the distal portion of the catheter body (33) is substantially encapsulated and constrained by the materials which comprise the catheter body (33).

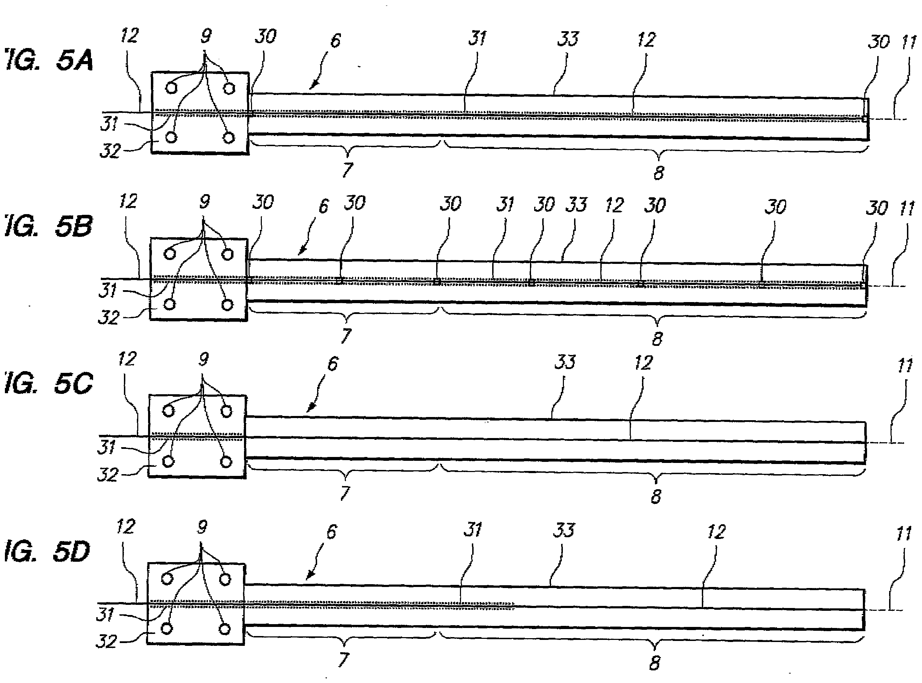

Figures 5A-5D depict embodiments analogous to those depicted in Figures 4A-D, with the exception that the fiber (12) is positioned substantially along the neutral axis of bending (11) of the catheter body (33), and in the embodiment of Figure 5B, there are seven constraints (30) as opposed to the three of the embodiment in Figure 4B.

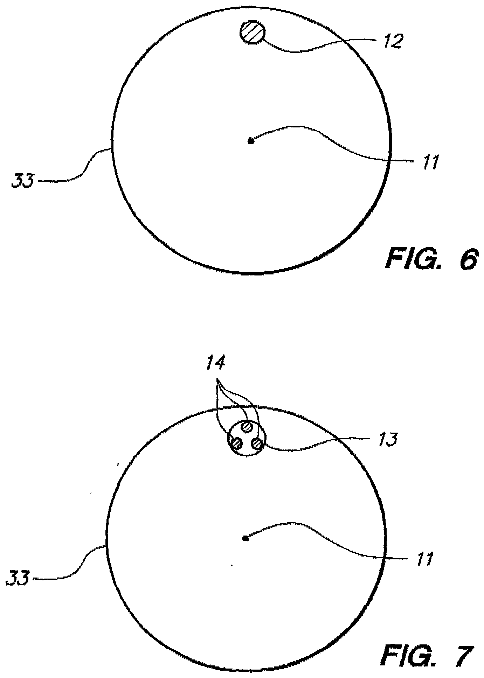

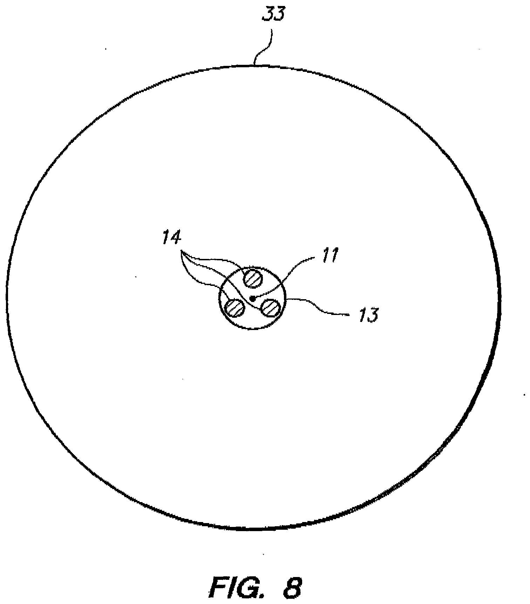

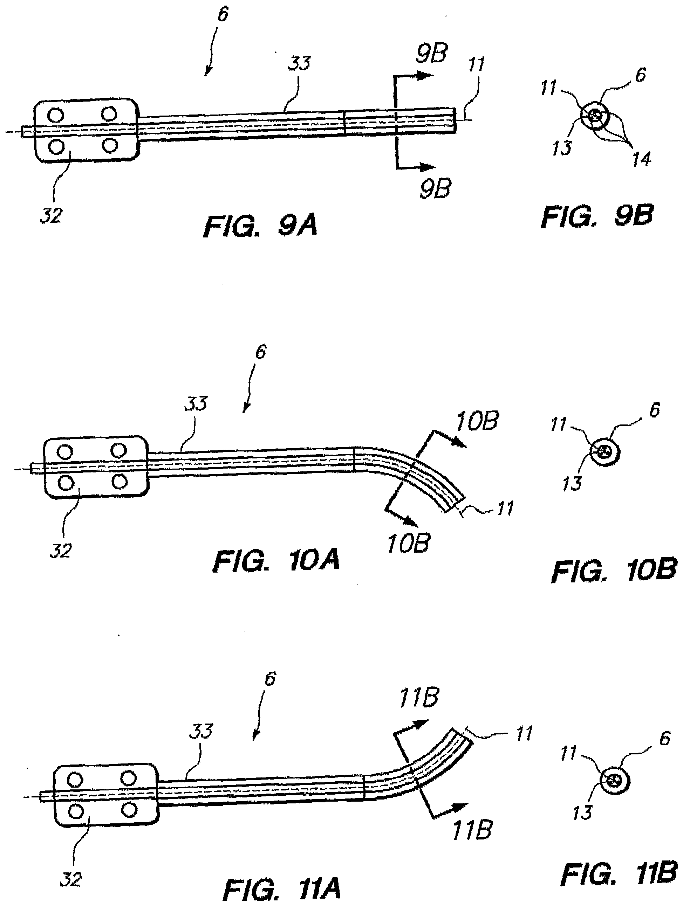

Referring to Figure 6, a cross section of a portion of the catheter body (33) of the configuration depicted in Figure 4C is depicted, to clearly illustrate that the fiber (12) is not placed concentrically with the neutral axis (11) of bending for the sample cross section. Figure 7 depicts a similar embodiment, wherein a multi-fiber bundle (13), such as those available from Luna Technologies, Inc., is positioned within the wall of the catheter rather than a single fiber as depicted in Figure 6, the fiber bundle (13) comprising multiple, in this embodiment three, individual (e.g., smaller) fibers or fiber cores (14). When a structure such as that depicted in Figure 7 is placed in bending in a configuration such as that depicted in Figure 3B or 3C, the most radially outward (from the neutral axis of bending (11)) of the individual fibers (14) experiences more compression or tension than the more radially inward fibers. Alternatively, in an embodiment such as that depicted in Figure 8, which shows a cross section of the catheter body (33) portion a configuration such as that depicted in Figure 5C, a multi-fiber bundle (13) is positioned coaxially with the neutral axis of bending (11) for the catheter (6), and each of three individual fibers (14) within the bundle (13) will experience different degrees of tension and/or compression in accordance with the bending or steering configuration of the subject catheter. For example, referring to Figures 9A and 9B (a cross section), at a neutral position, all three individual fibers (14) comprising the depicted bundle (13) may be in an unloaded configuration. With downward bending, as depicted in Figure 10A and 10B (a cross section), the lowermost two fibers comprising the bundle (13) may be configured to experience compression, while the uppermost fiber experiences tension. The opposite would happen with an upward bending scenario such as that depicted in Figures 11A and 11B (cross section).

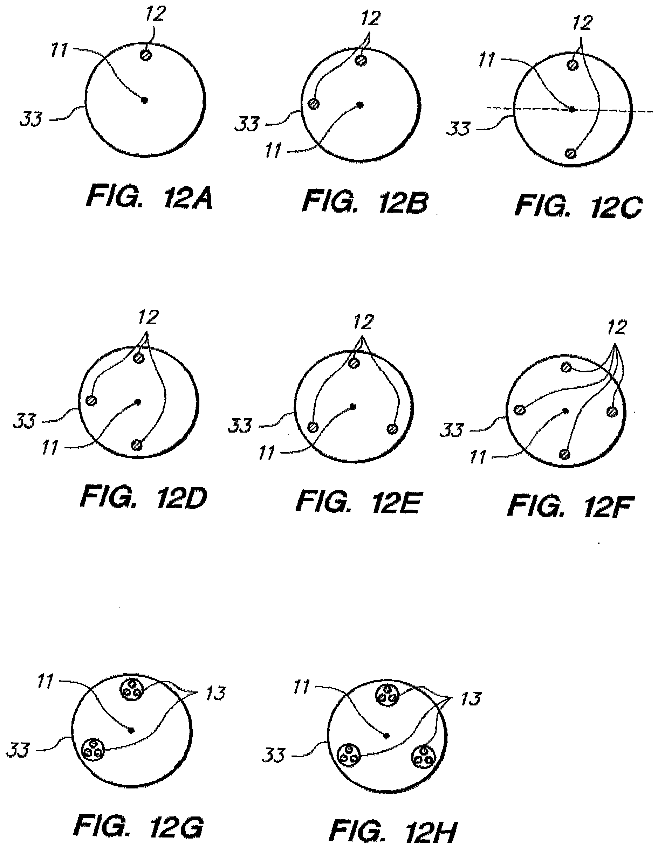

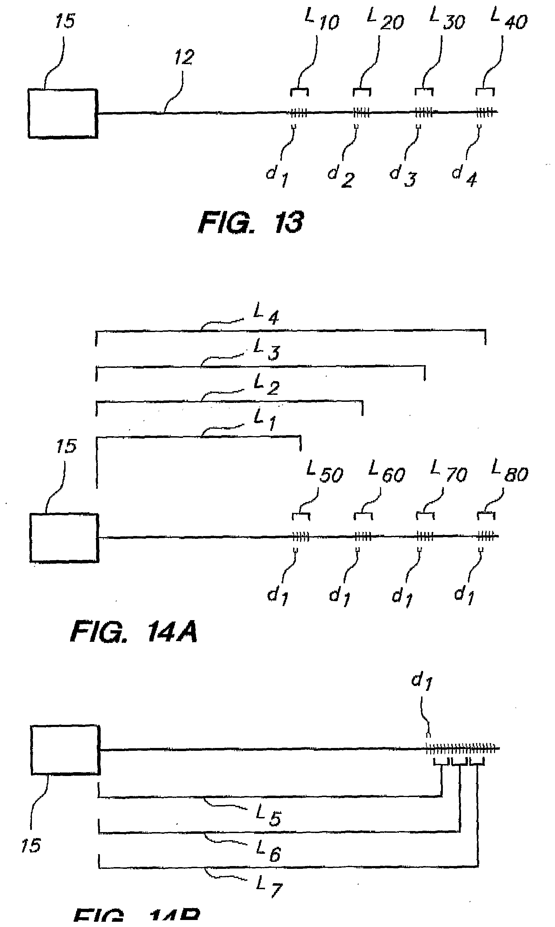

Indeed, various configurations may be employed, depending upon the particular application, such as those depicted in Figures 12A-12H. For simplicity, each of the cross sectional embodiments of Figures 12A-12H is depicted without reference to lumens adjacent the fibers, or constraints (i.e., each of the embodiments of Figures 12A-12H are depicted in reference to catheter body configurations analogous to those depicted, for example, in Figures 4C and 5C, wherein the fibers are substantially encapsulated by the materials comprising the catheter body (33); additional variations comprising combinations and permutations of constraints and constraining structures, such as those depicted in Figures 4A-5D, are within the scope of this invention. Figure 12A depicts an embodiment having one fiber (12). Figure 12B depicts a variation having two fibers (12) in a configuration capable of detecting tensions sufficient to calculate three-dimensional spatial deflection of the catheter portion. Figure 12C depicts a two-fiber variation with what may be considered redundancy for detecting bending about a bending axis such as that depicted in Figure 12C. Figures 12D and 12E depict three-fiber configurations configured for detecting three-dimensional spatial deflection of the subject catheter portion. Figure 12F depicts a variation having four fibers configured to accurately detect three-dimensional spatial deflection of the subject catheter portion. Figures 12G and 12H depict embodiments similar to 12B and 12E, respectively, with the exception that multiple bundles of fibers are integrated, as opposed to having a single fiber in each location. Each of the embodiments depicted in Figures 12A-12H, each of which depicts a cross section of an elongate instrument comprising at least one optical fiber, may be utilized to facilitate the determination of bending deflection, torsion, compression or tension, and/or temperature of an elongate instrument. Such relationships may be clarified in reference to Figures 13, 14A, and 14B.

In essence, the 3-dimensional position of an elongate member may be determined by determining the incremental curvature experienced along various longitudinal sections of such elongate member. In other words, if you know how much an elongate member has curved in space at several points longitudinally down the length of the elongate member, you can determine the position of the distal portion and more proximal portions in three-dimensional space by virtue of the knowing that the sections are connected, and where they are longitudinally relative to each other. Towards this end, variations of embodiments such as those depicted in Figures 12A-12H may be utilized to determine the position of a catheter or other elongate instrument in 3-dimensional space. To determine local curvatures at various longitudinal locations along an elongate instrument, fiber optic Bragg grating analysis may be utilized.

Referring to Figure 13, a single optical fiber (12) is depicted having four sets of Bragg diffraction gratings, each of which may be utilized as a local deflection sensor. Such a fiber (12) may be interfaced with portions of an elongate instrument, as depicted, for example, in Figures 12A-12H. A single detector (15) may be utilized to detect and analyze signals from more than one fiber. With a multi-fiber configuration, such as those depicted in Figures 12B-12H, a proximal manifold structure may be utilized to interface the various fibers with one or more detectors. Interfacing techniques for transmitting signals between detectors and fibers are well known in the art of optical data transmission. The detector is operatively coupled with a controller configured to determine a geometric configuration of the optical fiber and, therefore, at least a portion of the associated elongate instrument (e.g., catheter) body based on a spectral analysis of the detected reflected light signals. Further details are provided in Published US Patent Application 2006/0013523.

In the single fiber embodiment depicted in Figure 13, each of the diffraction gratings has a different spacing (d1, d2, d3, d4), and thus a proximal light source for the depicted single fiber and detector may detect variations in wavelength for each of the "sensor" lengths (L10, L20, L30, L40). Thus, given determined length changes at each of the "sensor" lengths (L10, L20, L30, L40), the longitudinal positions of the "sensor" lengths (L10, L20, L30, L40), and a known configuration such as those depicted in cross section in Figures 12A-12H, the deflection and/or position of the associated elongate instrument in space may be determined. One of the challenges with a configuration such as that depicted in Figure 13 is that a fairly broad band emitter and broad band tunable detector must be utilized proximally to capture length differentiation data from each of the sensor lengths, potentially compromising the number of sensor lengths that may be monitored, etc. Regardless, several fiber (12) and detector (15) configurations such as that depicted in Figure 13 may comprise embodiments such as those depicted in Figures 12A-12H to facilitate determination of three-dimensional positioning of an elongate medical instrument.

In another embodiment of a single sensing fiber, depicted in Figure 14A, various sensor lengths (L50, L60, L70, L80) may be configured to each have the same grating spacing, and a more narrow band source may be utilized with some sophisticated analysis, as described, for example, in "Sensing Shape - Fiber-Bragg-grating sensor arrays monitor shape at high resolution," SPIE's OE Magazine, September, 2005, pages 18-21, to monitor elongation at each of the sensor lengths given the fact that such sensor lengths are positioned at different positions longitudinally (L1, L2, L3, L4) away from the proximal detector (15). In another (related) embodiment, depicted in Figure 14B, a portion of a given fiber, such as the distal portion, may have constant gratings created to facilitate high-resolution detection of distal lengthening or shortening of the fiber. Such a constant grating configuration would also be possible with the configurations described in the aforementioned scientific journal article.

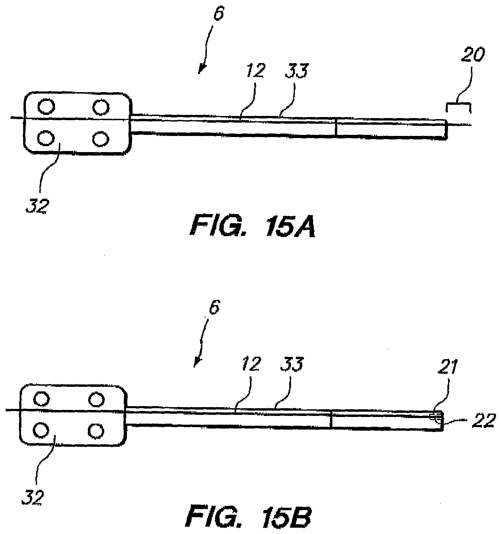

Referring to Figures 15A and 15B, temperature may be sensed utilizing Fiber-Bragg grating sensing in embodiments similar to those depicted in Figures 13 and 14A-B. Referring to Figure 15A, a single fiber protrudes beyond the distal tip of the depicted catheter (6) and is unconstrained, or at least less constrained, relative to other surrounding structures so that the portion of the depicted fiber is free to change in length with changes in temperature. With knowledge of the thermal expansion and contraction qualities of the small protruding fiber portion, and one or more Bragg diffraction gratings in such protruding portion, the changes in length may be used to extrapolate changes in temperature and thus be utilized for temperature sensing. Referring to Figure 15B, a small cavity (21) or lumen may be formed in the distal portion of the catheter body (33) to facilitate free movement of the distal portion (22) of the fiber (12) within such cavity (21)to facilitate temperature sensing distally without the protruding fiber depicted in Figure 15A.

The fibers in the embodiments depicted herein will provide accurate measurements of localized length changes in portions of the associated catheter or elongate instrument only if such fiber portions are indeed coupled in some manner to the nearby portions of the catheter or elongate instrument. In one embodiment, it is desirable to have the fiber or fibers intimately coupled with or constrained by the surrounding instrument body along the entire length of the instrument, with the exception that one or more fibers may also be utilized to sense temperature distally, and may have an unconstrained portion, as in the two scenarios described in reference to Figures 15A and 15B. In one embodiment, for example, each of several deflection-sensing fibers may terminate in a temperature sensing portion, to facilitate position determination and highly localized temperature sensing and comparison at different aspects of the distal tip of an elongate instrument. In another embodiment, the proximal portions of the fiber(s) in the less bendable catheter sections are freely floating within the catheter body, and the more distal/bendable fiber portions intimately coupled, to facilitate high-precision monitoring of the bending within the distal, more flexible portion of the catheter or elongate instrument.

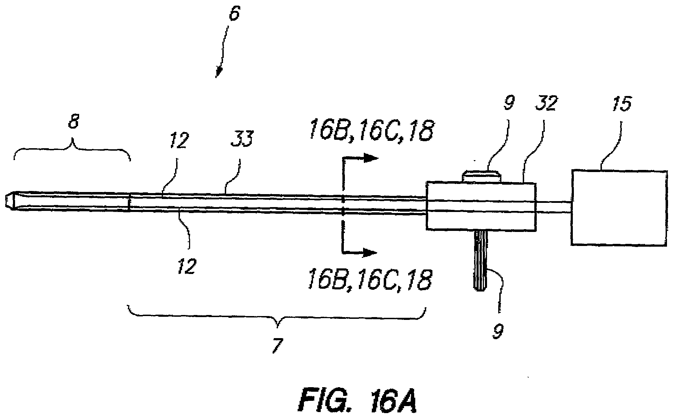

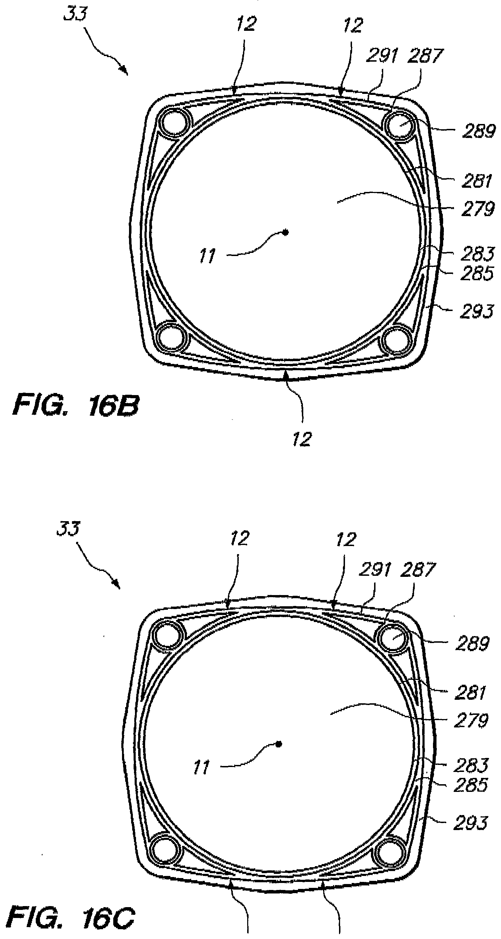

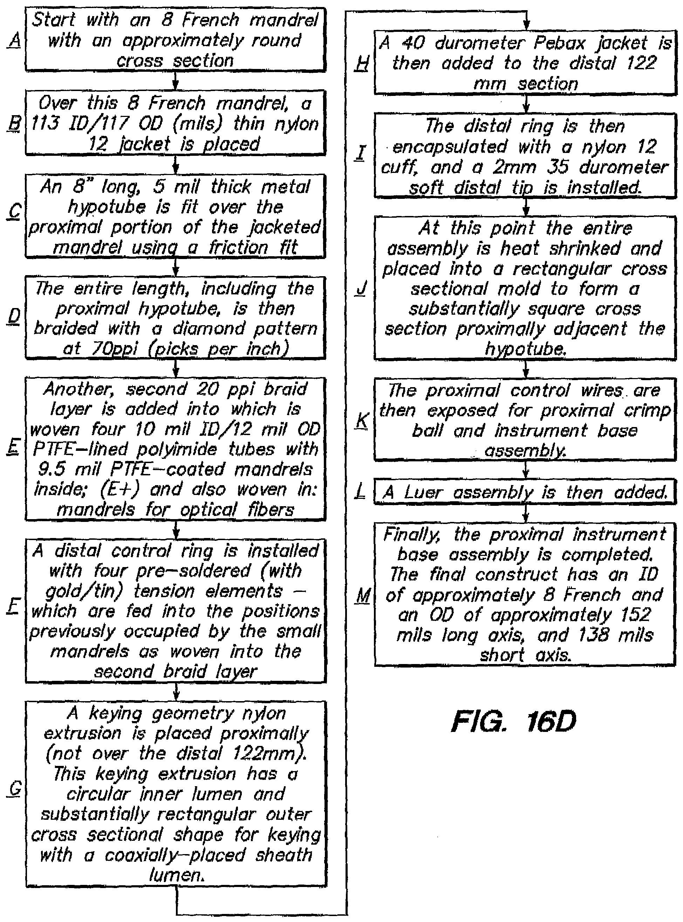

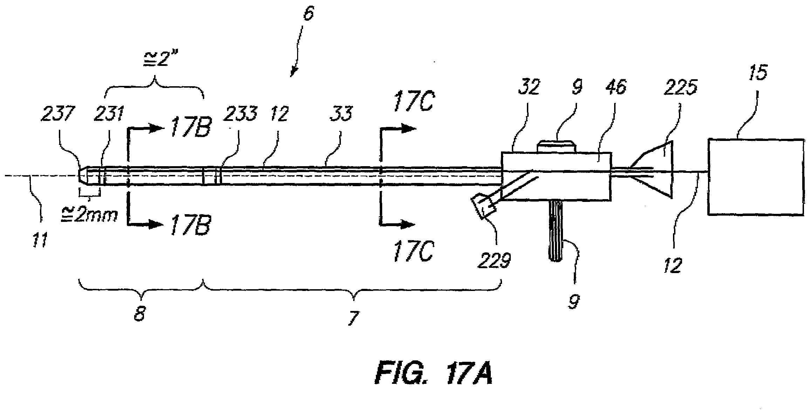

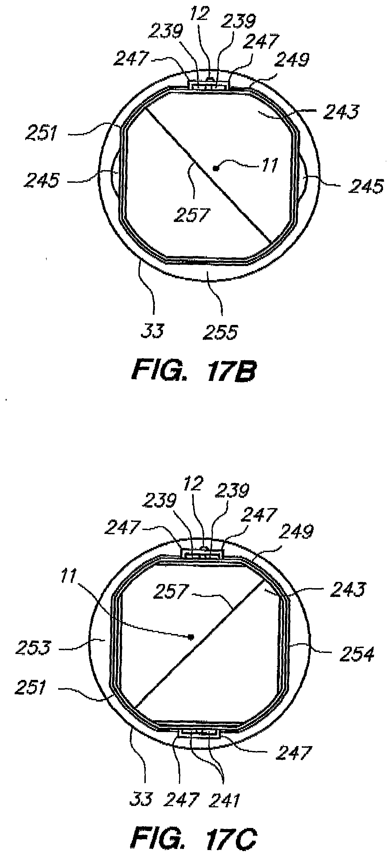

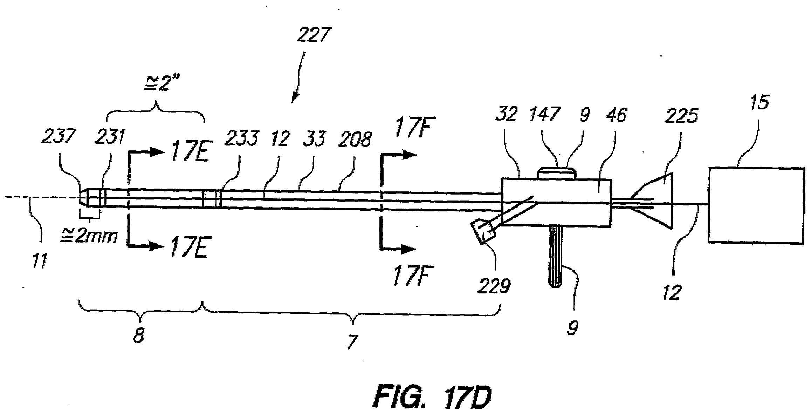

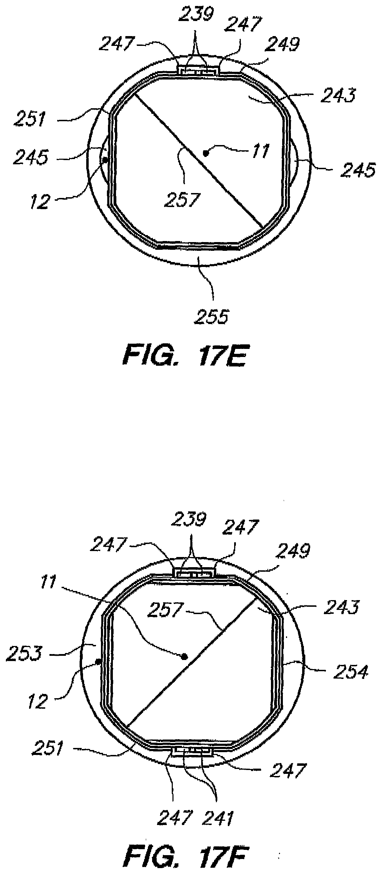

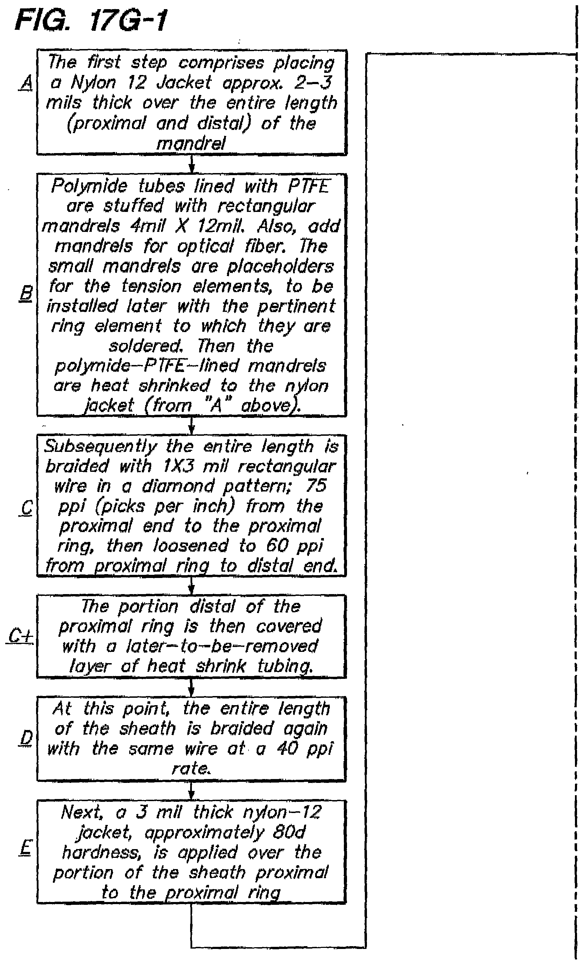

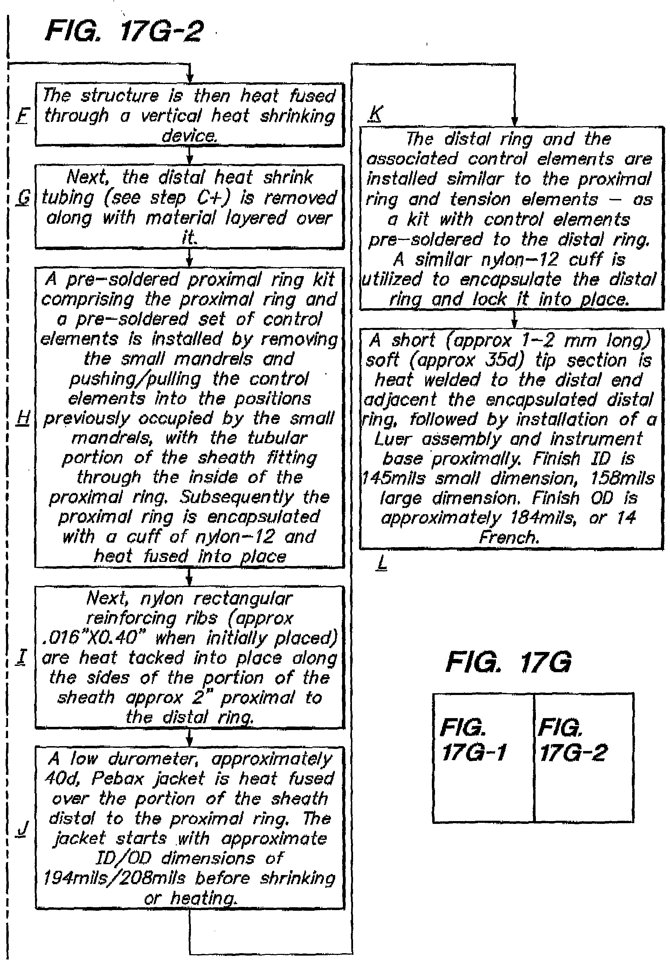

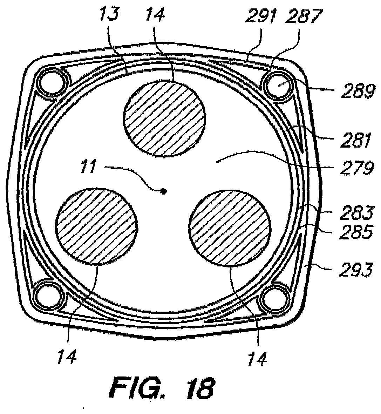

Referring to Figures 16A, 16B, and 16D, a catheter-like robotic guide instrument integration embodiment is depicted. U.S. Patent Application serial number 11/176,598, from which these drawings (along with Figures 17 and 18) have been taken and modified. Figures 16A and 16B show an embodiment with three optical fibers (12) and a detector (15) for detecting catheter bending and distal tip position. Figure 16C depicts and embodiment having four optical fibers (12) for detecting catheter position. Figure 16D depicts an integration to build such embodiments. As shown in Figure 16D, in step "E+", mandrels for optical fibers are woven into a braid layer, subsequent to which (step "F") Bragg-grated optical fibers are positioned in the cross sectional space previously occupied by such mandrels (after such mandrels are removed). The geometry of the mandrels relative to the fibers selected to occupy the positions previously occupied by the mandrels after the mandrels are removed preferably is selected based upon the level of constraint desired between the fibers (12) and surrounding catheter body (33) materials. For example, if a highly-constrained relationship, comprising substantial encapsulation, is desired, the mandrels will closely approximate the size of the fibers. If a more loosely-constrained geometric relationship is desired, the mandrels may be sized up to allow for relative motion between the fibers (12) and the catheter body (33) at selected locations, or a tubular member, such as a polyimide or PTFE sleeve, may be inserted subsequent to removal of the mandrel, to provide a "tunnel" with clearance for relative motion of the fiber, and/or simply a layer of protection between the fiber and the materials surrounding it which comprise the catheter or instrument body (33). Similar principles may be applied in embodiments such as those described in reference to Figures 17A-17G.

Referring to Figures 17A-F, two sheath instrument integrations are depicted, each comprising a single optical fiber (12). Figure 17G depicts an integration to build such embodiments. As shown in Figure 16D, in step "B", a mandrel for the optical fiber is placed, subsequent to which (step "K") a Bragg-grated optical fiber is positioned in the cross sectional space previously occupied by the mandrel (after such mandrel is removed).

Referring to Figure 18, in another embodiment, a bundle (13) of fibers (14) may be placed down the working lumen of an off-the-shelf robotic catheter (guide or sheath instrument type) such as that depicted in Figure 18, and coupled to the catheter in one or more locations, with a selected level of geometric constraint, as described above, to provide 3-D spatial detection.

Tension and compression loads on an elongate instrument may be detected with common mode deflection in radially-outwardly positioned fibers, or with a single fiber along the neutral bending axis. Torque may be detected by sensing common mode additional tension (in addition, for example, to tension and/or compression sensed by, for example, a single fiber coaxial with the neutral bending axis) in outwardly-positioned fibers in configurations such as those depicted in Figures 12A-H.

In another embodiment, the tension elements utilized to actuate bending, steering, and/or compression of an elongate instrument, such as a steerable catheter, may comprise optical fibers with Bragg gratings, as compared with more conventional metal wires or other structures, and these fiber optic tension elements may be monitored for deflection as they are loaded to induce bending/steering to the instrument. Such monitoring may be used to prevent overstraining of the tension elements, and may also be utilized to detect the position of the instrument as a whole, as per the description above.

## Claims

1. A medical instrument system, comprising:
an optical fiber (12), the optical fiber (12) including one or more Bragg gratings;
an elongate catheter body (33) comprising a lumen (31), the optical fiber (12) being disposed in the lumen (31);
a detector (15) operably coupled to a proximal end of the optical fiber (12) and configured to detect respective light signals reflected by the one or more Bragg gratings; and
a controller operatively coupled to the detector (15) and configured to determine a geometric configuration of at least a portion of the elongate catheter body (33) based on a spectral analysis of the detected reflected portions of the light signals;
**characterized in that**
the optical fiber (12) is coupled in a constrained manner to the elongate catheter body (33),
wherein the system comprises constraints (30) prohibiting axial or longitudinal motion of the optical fiber (12) relative to the lumen (31) at each location of the constraints (30), and
wherein the system comprises further constraints (30) configured to guide the optical fiber (12) and to allow relative longitudinal or axial motion of the optical fiber (12) relative to the lumen (31) at each location of the further constraints (30).

2. The medical instrument system of claim 1, wherein the elongate catheter body (33) is flexible.

3. The medical instrument system of claims 1 or 2, wherein the elongate catheter body (33) is robotically controlled.

4. The medical instrument system of claims 1 or 2, wherein the elongate catheter body (33) is manually controlled.

5. The medical instrument system of any of claims 1-4, further comprising a reference reflector coupled to the optical fiber (12) in an operable relationship with the one or more Bragg gratings.

6. The medical instrument system of any of claims 1-5, the detector (15) comprising a frequency domain reflectometer.

7. The medical instrument system of any of claims 1-6, wherein the optical fiber (12) comprises multiple fiber cores, each core including one or more Bragg gratings.

8. The medical instrument system of claim 7, each optical fiber core comprising a plurality of spaced apart Bragg gratings.

9. The medical instrument system of any of claims 1-6, the optical fiber (12) comprising a plurality of spaced apart Bragg gratings.

10. The medical instrument system of any of claims 1-9, the elongate catheter body (33) having a neutral axis of bending, the optical fiber (12) being coupled to the elongate catheter body (33) so as to be substantially aligned with the neutral axis of bending regardless of bending of the elongate catheter body (33).

11. The medical instrument system of any of claims 1-9, the elongate catheter body (33) having a neutral axis of bending, the optical fiber (12) being coupled to the elongate catheter body (33) so as to remain substantially parallel to, but not aligned with, the neutral axis of bending regardless of bending of the elongate catheter body (33).

## Patentansprüche

1. Ein Medizininstrumentsystem umfassend:
eine optische Faser (12), wobei die optische Faser (12) ein oder mehrere Bragg-Gitter umfasst;
einen länglichen Katheterkörper (33) umfassend ein Lumen (31), wobei die optische Faser (12) in dem Lumen (31) angeordnet ist;
einen Detektor (15) der betriebsbereit mit einem proximalen Ende der optischen Faser (12) gekoppelt ist und dazu konfiguriert ist, Lichtsignale zu detektieren, die von dem einen oder den mehreren Bragg-Gittern reflektiert worden sind; und
einen Controller, der betriebsbereit mit dem Detektor (15) gekoppelt ist und dazu konfiguriert ist, eine geometrische Konfiguration von zumindest einem Teil des länglichen Katheterkörpers (33) auf der Grundlage einer Spektralanalyse der detektierten reflektierten Teile der Lichtsignale zu bestimmen;
**dadurch gekennzeichnet, dass**
die optische Faser (12) in einer beschränkten Weise mit dem länglichen Katheterkörper (33) gekoppelt ist, wobei das System Beschränkungen (30) umfasst, die eine axiale oder longitudinale Bewegung der optischen Faser (12) relativ zu dem Lumen (31) an jedem Ort der Beschränkungen (30) unterbinden, und wobei das System weitere Beschränkungen (30) umfasst, die dazu konfiguriert sind, die optische Faser (12) zu führen und eine relative longitudinale oder axiale Bewegung der optischen Faser (12) relativ zu dem Lumen (31) an jedem Ort der weiteren Beschränkungen (30) zu erlauben.

2. Das Medizininstrumentsystem von Anspruch 1, wobei der längliche Katheterkörper (33) flexibel ist.

3. Das Medizininstrumentsystem von Anspruch 1 oder 2, wobei der längliche Katheterkörper (33) robotergesteuert ist.

4. Das Medizininstrumentsystem von Anspruch 1 oder 2, wobei der längliche Katheterkörper (33) manuell gesteuert ist.

5. Das Medizininstrumentsystem von irgendeinem der Ansprüche 1 - 4, weiter umfassend einen Referenzreflektor, der mit der optischen Faser (12) in einer betriebsbereiten Beziehung mit dem einem oder den mehreren Bragg-Gittern gekoppelt ist.

6. Das Medizininstrumentsystem von irgendeinem der Ansprüche 1 - 5, wobei der Detektor (15) ein Frequenzbereichsreflektometer umfasst.

7. Das Medizininstrumentsystem von irgendeinem der Ansprüche 1 - 6, wobei die optische Faser (12) mehrere Faserseelen umfasst, wobei jede Seele ein oder mehrere Bragg-Gitter umfasst.

8. Das Medizininstrumentsystem von Anspruch 7, wobei jede optische Faserseele eine Mehrzahl von voneinander beabstandeten Bragg-Gittern umfasst.

9. Das Medizininstrumentsystem von irgendeinem der Ansprüche 1-6, wobei die optische Faser (12) eine Mehrzahl von voneinander beabstandeten Bragg-Gittern umfasst.

10. Das Medizininstrumentsystem von irgendeinem der Ansprüche 1-9, wobei der längliche Katherterkörper (33) eine neutrale Biegeachse hat, wobei die optische Faser (12) mit dem länglichen Katherterkörper (33) gekoppelt ist, um so, ungeachtet einer Biegung des länglichen Katherterkörpers (33), im Wesentlichen entlang der neutralen Biegeachse ausgerichtet zu sein.

11. Das Medizininstrumentsystem von irgendeinem der Ansprüche 1-9, wobei der längliche Katherterkörper (33) eine neutrale Biegeachse hat, wobei die optische Faser (12) mit dem länglichen Katherterkörper (33) gekoppelt ist, um so, ungeachtet einer Biegung des länglichen Katherterkörpers (33), im Wesentlichen parallel zu aber nicht ausgerichtet entlang der neutralen Biegeachse zu bleiben.

## Revendications

1. Système d'instrumentation médicale comprenant :
une fibre optique (12), la fibre optique (12) englobant un ou plusieurs réseaux de Bragg ;
un corps de cathéter allongé (33) comprenant une lumière (31), la fibre optique (12) étant disposée dans la lumière (31) ;
un détecteur (15) couplé de manière opérationnelle à une extrémité proximale de la fibre optique (12) et configuré pour détecter des signaux lumineux respectifs réfléchis par lesdits un ou plusieurs réseaux de Bragg ; et
un contrôleur couplé de manière opérationnelle au détecteur (15) et configuré pour déterminer une configuration géométrique d'au moins une portion du corps de cathéter allongé (33) en se basant sur une analyse spectrale des portions réfléchies détectées des signaux lumineux ;
**caractérisé en ce que**
la fibre optique (12) est couplée d'une manière contraignante au corps de cathéter allongé (33) ;
dans lequel le système comprend des restrictions (30) empêchant un mouvement axial ou longitudinal de la fibre optique (12) par rapport à la lumière (31) à chaque endroit occupé par les restrictions (30) ; et
dans lequel le système comprend des restrictions supplémentaires (30) configurées pour guider la fibre optique (12) et pour permettre un mouvement longitudinal ou axial relatif de la fibre optique (12) par rapport à la lumière (31) à chaque endroit occupé par les restrictions (30).

2. Système d'instrumentation médicale selon la revendication 1, dans lequel le corps de cathéter allongé (33) est flexible.

3. Système d'instrumentation médicale selon la revendication 1 ou 2, dans lequel le corps de cathéter allongé (33) est commandé par un robot.

4. Système d'instrumentation médicale selon la revendication 1 ou 2, dans lequel le corps de cathéter allongé (33) fait l'objet d'une commande manuelle.

5. Système d'instrumentation médicale selon l'une quelconque des revendications 1 à 4, comprenant en outre un réflecteur de référence couplé à la fibre optique (12) dans une relation opérationnelle avec lesdits un ou plusieurs réseaux de Bragg.

6. Système d'instrumentation médicale selon l'une quelconque des revendications 1 à 5, dans lequel le détecteur (15) comprend un réflectomètre dans le domaine de fréquence.

7. Système d'instrumentation médicale selon l'une quelconque des revendications 1 à 6, dans lequel la fibre optique (12) comprend plusieurs coeurs de fibres, chaque coeur englobant un ou plusieurs réseaux de Bragg.

8. Système d'instrumentation médicale selon la revendication 7, dans lequel chaque coeur de fibre optique comprend plusieurs réseaux de Bragg espacés les uns des autres.

9. Système d'instrumentation médicale selon l'une quelconque des revendications 1 à 6, dans lequel la fibre optique (12) comprend plusieurs réseaux de Bragg espacés les uns des autres.

10. Système d'instrumentation médicale selon l'une quelconque des revendications 1 à 9, dans lequel le corps de cathéter allongé (33) possède un axe neutre de flexion, la fibre optique (12) étant couplée au corps de cathéter allongé (33) de manière à être essentiellement mise en alignement avec l'axe neutre de flexion indépendamment de la flexion du corps de cathéter allongé (33).

11. Système d'instrumentation médicale selon l'une quelconque des revendications 1 à 9, dans lequel le corps de cathéter allongé (33) possède un axe neutre de flexion, la fibre optique (12) étant couplée au corps de cathéter allongé (33) de manière à rester essentiellement en parallèle, mais non en alignement, avec l'axe neutre de flexion indépendamment de la flexion du corps de cathéter allongé (33).
